# EUROPEAN PATENT SPECIFICATION

(11) **EP 1 543 776 B1**
(45) Date of publication and mention of the grant of the patent: **11.01.2012**
(21) Application number: 03798404.4
(22) Date of filing: 12.09.2003
(51) Int. Cl.: A61B 8/12

(54) **ULTRASONOGRAPH**
ULTRASCHALLGERÄT
ECHOGRAPHE

(30) Priority: 27.09.2002 JP 2002283803
(43) Date of publication of application: 22.06.2005
(73) Proprietor: Olympus Corporation, Tokyo 151-0072 (JP)
(72) Inventor: OKUNO, Yoshiyuki, Fussa-shi, Tokyo 197-0023 (JP)
(74) Representative: von Hellfeld, Axel
(86) International application number: PCT/JP2003/011694
(87) International publication number: WO 2004/028374

(56) References cited:
- EP-A- 0 894 473
- DE-A1- 19 751 761
- JP-A- 7 184 906
- JP-A- 8 257 028
- JP-A- 11 047 133
- JP-A- 11 047 133
- JP-A- 11 123 187
- JP-A- 2000 023 979
- JP-A- 2000 023 980
- JP-A- 2000 023 980
- JP-A- 2001 017 433
- JP-A- 2002 263 101
- US-A- 5 335 663

## Description

The present invention relates to an ultrasonic diagnosis apparatus in accordance with the preamble of claims 1 and 8, respectively.

Such an ultrasonic diagnosis apparatus is known from EP 0 894 473 A2 which shows an ultrasonic diagnosis apparatus having the capability of superimposing a map on an image obtained by an ultrasonic wave transmitting/receiving means.

### Background Art

One of ultrasonic diagnosis apparatus obtains a living body tomogram by irradiating ultrasonic pulses into a living body and receiving waves reflected from the living body tissue. Since the ultrasonic diagnosis apparatus can be used for the diagnosis of the inside of a living body noninvasively, the ultrasonic diagnosis apparatus is widely used for the external obstetric and gynecologic examinations.

In order to easily identify a contact position of an ultrasonic contact in the ultrasonic diagnosis apparatus, Japanese Unexamined Patent Application Publication No. 60-66735 discloses a diagnosed part displaying method for an ultrasonic diagnosis apparatus for displaying a sample three-dimensionally with the contour and multiple ellipses and displaying the position of an ultrasonic contact.

Furthermore, Japanese Unexamined Patent Application Publication No. 10-151131 discloses a method for displaying a CT image and an MRI image in accordance with the position being scanned by an ultrasonic contact instead of the display of a sample with an abstract body mark including the contour and multiple ellipses.

The positional relationship between an ultrasonic probe and a human body can be identified from a simple display of an object with the contour and multiple ellipses as disclosed in Japanese Unexamined Patent Publication Application No. 60-66735. However, the positional relationship between an organ being actually diagnosed and a probe is difficult for operators to understand.

Furthermore, an ultrasonic diagnosis apparatus connecting to a long and narrow, flexible ultrasonic endoscope to be inserted into an object does not allow operators to visually check the state of the ultrasonic endoscope. Therefore, the part being observed in the object is more difficult to identify, which is a problem, in comparison with an ultrasonic probe used in an external ultrasonic diagnosis apparatus disclosed in Japanese Unexamined Patent Application Publication No. 10-151131. Therefore, moving a tomogram toward a concerned area so as to render a desired section is very difficult in the ultrasonic endoscope, which significantly hinders the spread of ultrasonic endoscopes.

Also, an oval and spherical ultrasonic sonde easily swallowable from the mouth cavity, for example, of an object is connected to the ultrasonic diagnosis apparatus, the operator cannot visually check the state of the ultrasonic sonde. Therefore, the part being observed of the object is difficult to identify, which is another problem.

The object underlying the present invention is to ease for a user of the ultrasonic diagnosis apparatus the understanding of the ultrasonic image.

Alternative solutions of this problem according to the present invention are described in claims 1 and 8, respectively.

Preferred embodiments of the invention are described in the dependent claims.

### Disclosure of Invention

In the following description of several embodiments the embodiments nos. 1 and 2 serve for illustrative purposes only, whereas the claimed invention is described, in particular, in connection with the third embodiment and the following embodiments.

The other characteristics and advantages of the invention will be sufficiently apparent from the descriptions below.

### Brief Description of the Drawings

Figs. 1 to 3 relate to a first embodiment of the present invention;
Fig. 1 is a construction diagram showing a system configuration of an ultrasonic diagnosis apparatus;
Fig. 2 is a diagram showing a state where a mechanical scan type ultrasonic endoscope is being inserted to the body cavity;
Fig. 3 is a diagram for describing an operation of the ultrasonic diagnosis apparatus in Fig. 1;
Figs. 4 to 9 relate to a second embodiment of the invention;

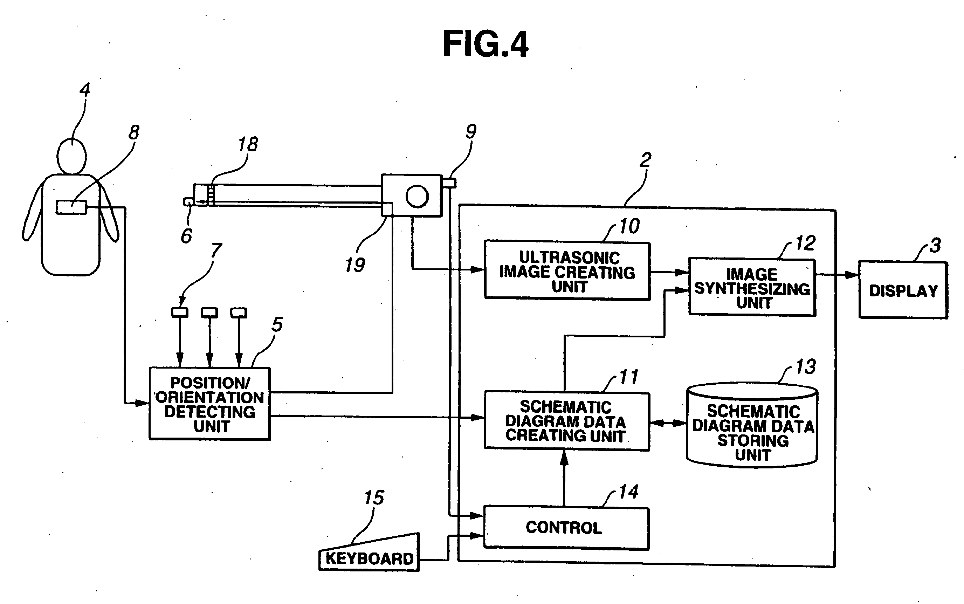

Fig. 4 is a construction diagram showing a system construction of an ultrasonic diagnosis apparatus;

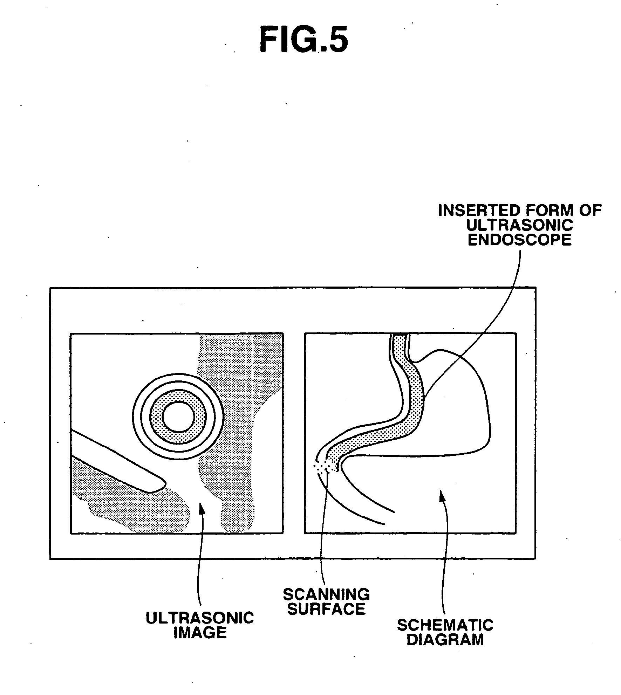

Fig. 5 is a diagram for describing an operation of the ultrasonic diagnosis apparatus in Fig. 4;

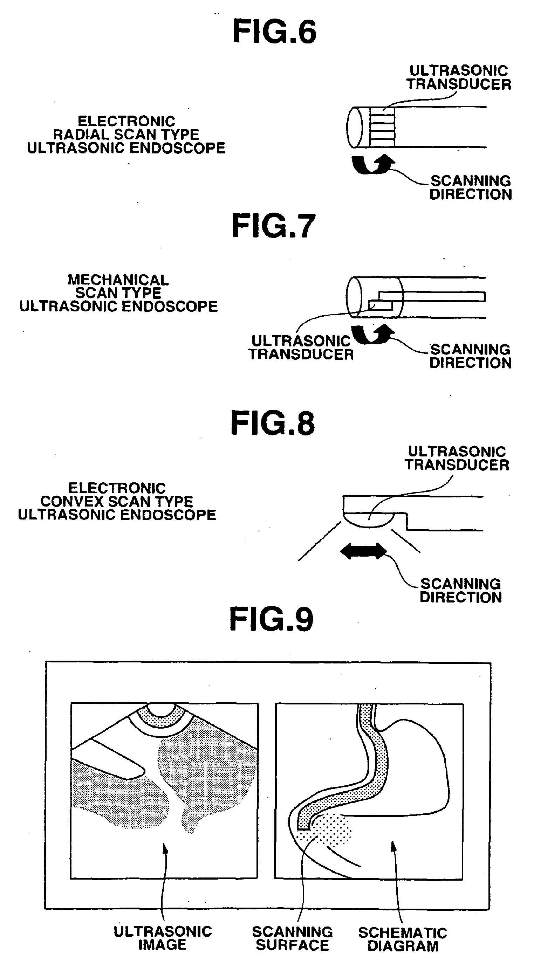

Fig. 6 is a diagram showing an arrangement of an ultrasonic transducer of an electronic radial scan type ultrasonic endoscope and an ultrasonic scanning surface;
Fig. 7 is a diagram showing an arrangement of an ultrasonic transducer of a mechanical scan type ultrasonic endoscope and an ultrasonic scanning surface;
Fig. 8 is a diagram showing an arrangement of an ultrasonic transducer of an electronic convex scan type ultrasonic endoscope and an ultrasonic scanning surface;
Fig. 9 is a diagram for describing an operation of an ultrasonic diagnosis apparatus in the electronic convex scan type ultrasonic endoscope in Fig. 8;

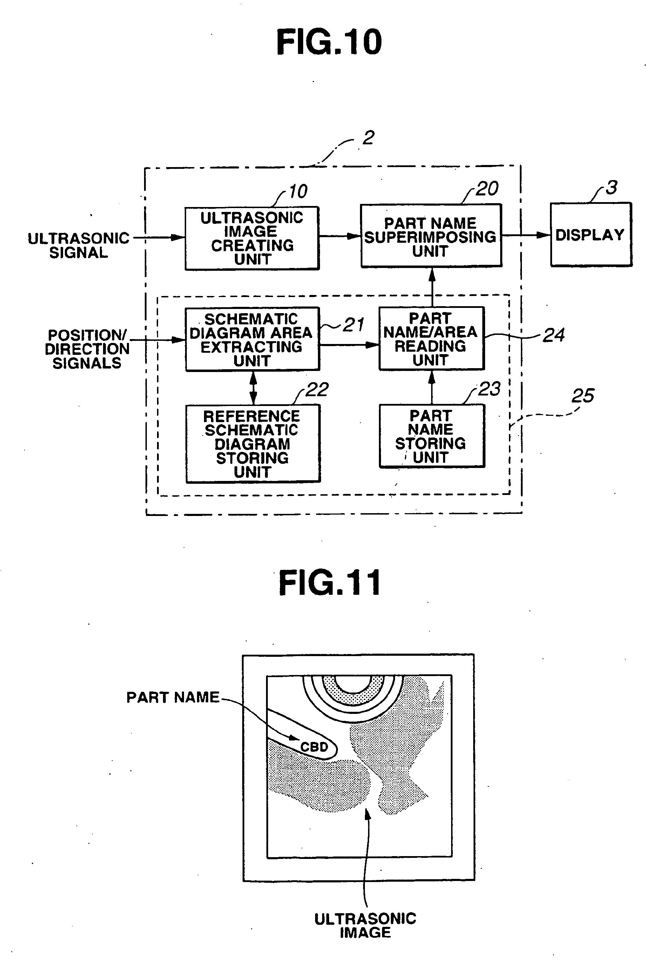

Figs. 10 to 15 relate to a third embodiment of the present invention;
Fig. 10 is a construction diagram showing a construction of an ultrasonic diagnosis apparatus;
Fig. 11 is a diagram for describing an operation of the ultrasonic diagnosis apparatus in Fig. 10;

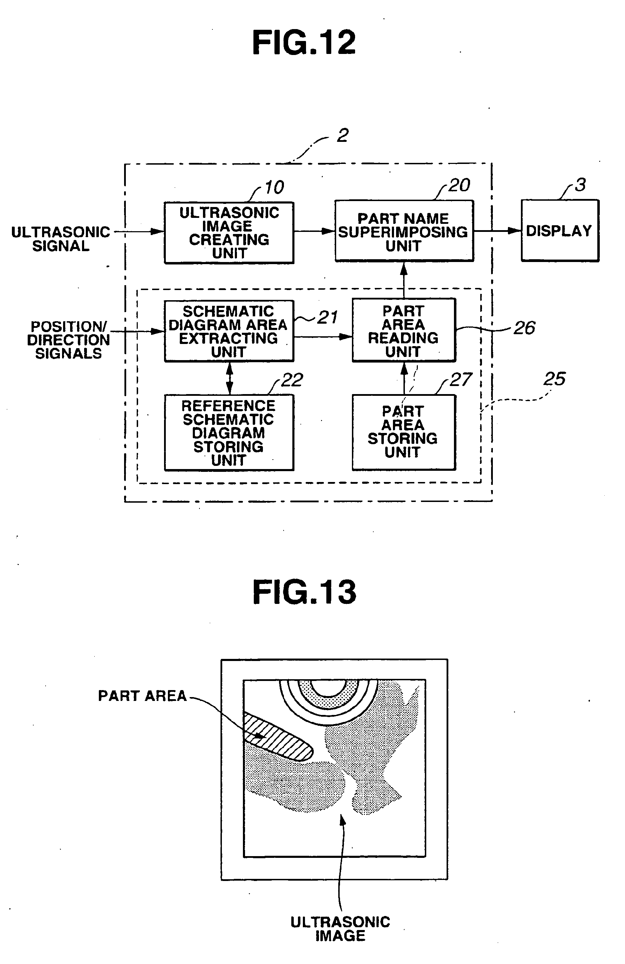

Fig. 12 is a construction diagram showing a construction of a first variation example of the ultrasonic diagnosis apparatus in Fig. 10;
Fig. 13 is a diagram for describing an operation of the first variation example of the ultrasonic diagnosis apparatus in Fig. 12;

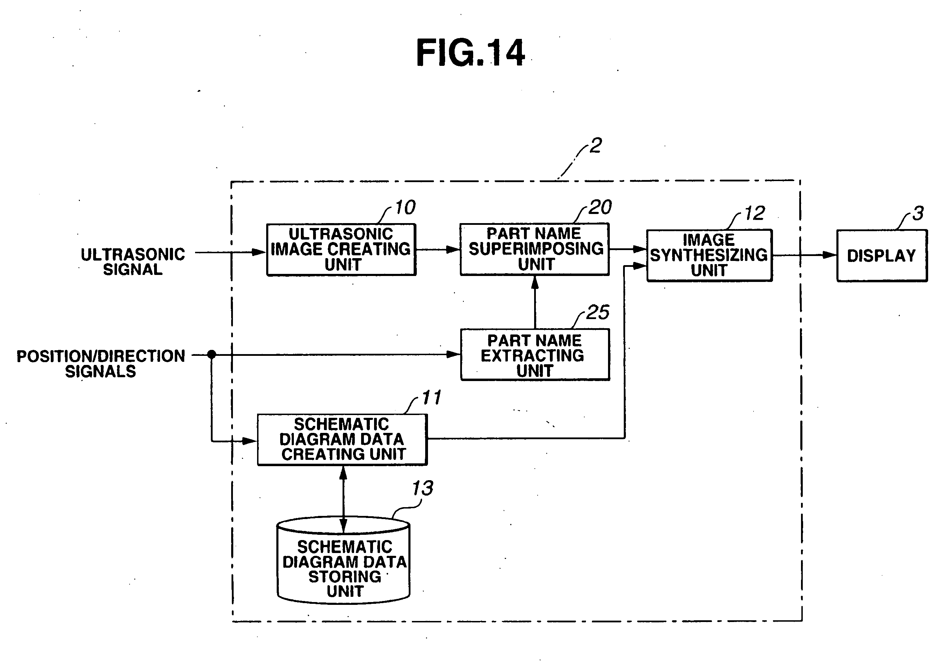

Fig. 14 is a construction diagram showing a construction of a second variation example of the ultrasonic diagnosis apparatus in Fig. 10;

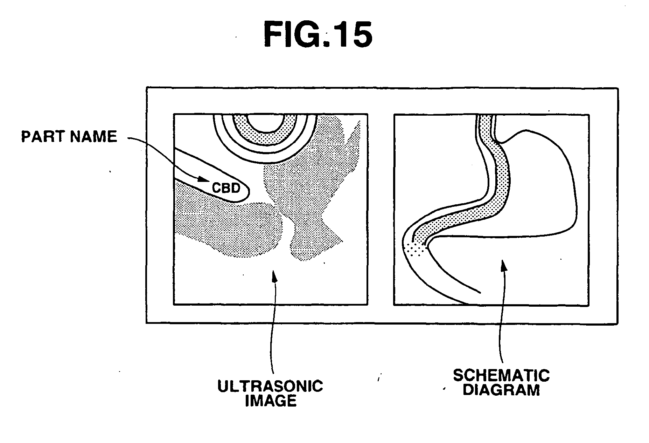

Fig. 15 is a diagram for describing an operation of a second variation example of the ultrasonic diagnosis apparatus in Fig. 14;

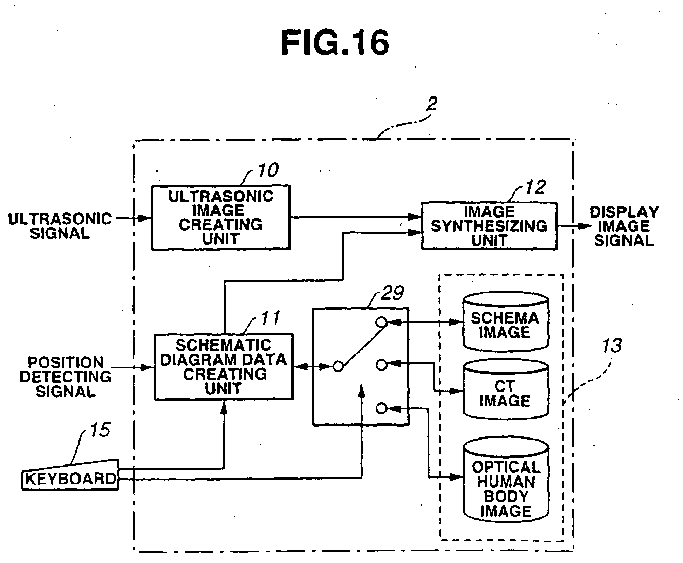

Fig. 16 is a construction diagram showing a construction of an ultrasonic diagnosis apparatus according to a fourth embodiment of the invention;

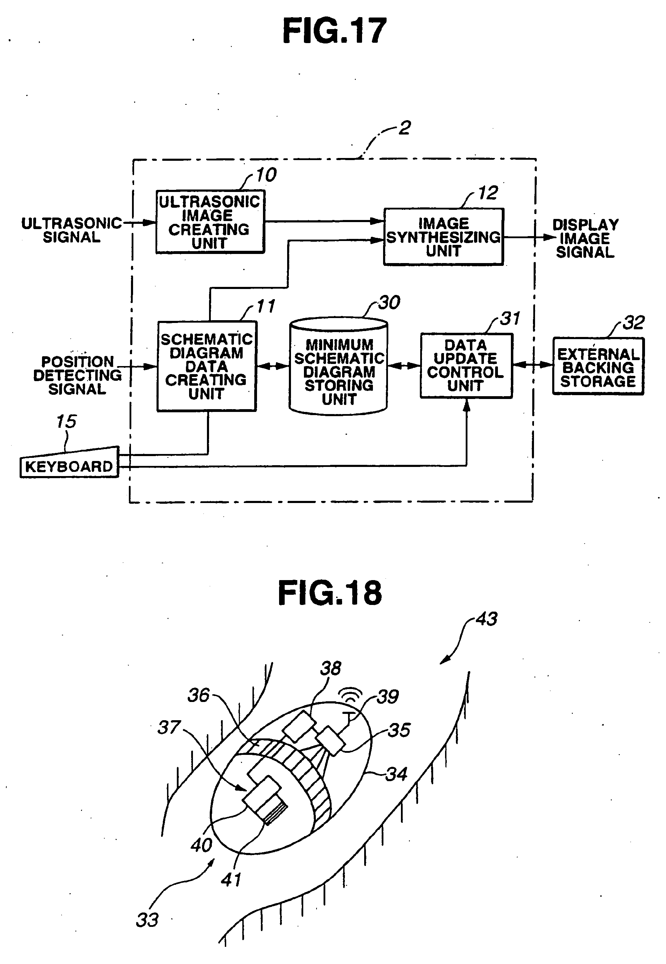

Fig. 17 is a construction diagram showing a construction of an ultrasonic diagnosis apparatus according to a fifth embodiment of the invention; and
Fig. 18 is a construction diagram showing a construction of a capsule type ultrasonic sonde according to a sixth embodiment of the invention.

### Best Mode for Carrying Out the Invention

The present invention will be described in detail with reference to appended drawings.

### First Embodiment

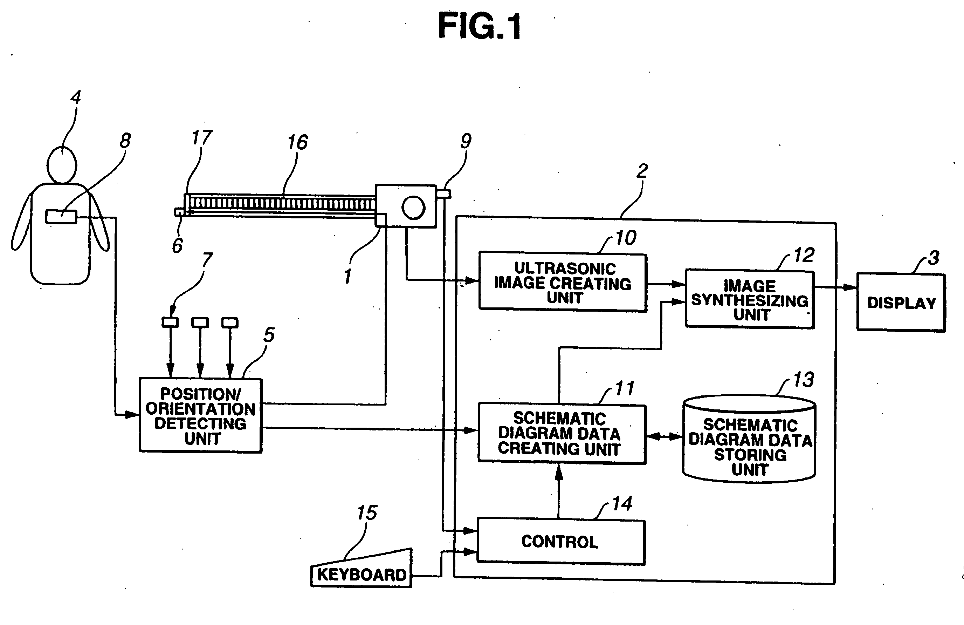

Fig. 1 includes a mechanical scan type ultrasonic endoscope 1, an ultrasonic diagnosis apparatus 2, a display 3, an object 4, a position/orientation detecting unit 5 (as an ultrasonic scan position detecting unit), a send coil 6, a receive coil 7, an attitude detecting unit 8, a scope SW 9, an ultrasonic image creating unit 10, a schematic diagram data creating unit 11, an image synthesizing unit 12, a schematic diagram data storing unit 13, a control 14, a keyboard 15, a shaft 16, and an ultrasonic transducer 17 (as an ultrasonic sending/receiving unit).

A system construction according to this embodiment will be described which uses the mechanical scan type ultrasonic endoscope 1 to make use of a magnetic field for detecting the position of the mechanical scan type ultrasonic endoscope 1.

Fig. 1 shows a system construction of an ultrasonic diagnosis apparatus according to this embodiment using a magnetic field for position detection.

In order to detect a ultrasonic scan position of the mechanical scan type ultrasonic endoscope 1, the send coil 6 for generating a magnetic field is implemented at the inserting end of the mechanical scan type ultrasonic endoscope 1. Signals generated by the magnetic field by the implemented send coil 6 are output from the position/orientation detecting unit 5. The position/orientation detection unit 5 has the receive coil 7 for receiving the magnetic field from the send coil 6 implemented in the mechanical scan type ultrasonic endoscope 1. Furthermore, signals from the attitude detecting unit 8 attached to the object 4 for detecting the attitude of the object 4 are input to the position/orientation detecting unit 5.

Thus, the position/orientation detecting unit 5 outputs to the ultrasonic diagnosis apparatus 2 signals indicating the attitude of the object 4 and signals indicating the ultrasonic scan position of the mechanical scan type ultrasonic endoscope 1.

In the ultrasonic diagnosis apparatus 2 according to this embodiment, the ultrasonic transducer 17 at the inserting end is mechanically rotated by the shaft 16 of the mechanical scan type ultrasonic endoscope 1. Thus, ultrasonic signals are scanned circumferentially about the shaft 16. Through this operation, the ultrasonic image creating unit 10 creates an ultrasonic image from the obtained ultrasonic signals.

On the other hand, the schematic diagram data creating unit 11 extracts schematic diagram data to be read from the schematic diagram data storing unit 13 from attitude position signals indicating the attitude of the object 4 obtained from the position/orientation detecting unit 5 and the position for scanning ultrasonic wave in the mechanical scan type ultrasonic endoscope 1. In order to detect the position of the mechanical scan type ultrasonic endoscope 1, a reference position for starting the detection must be specified.

The reference position may be specified by turning on the keyboard 15 or the scope SW 9 when the inserting end of the mechanical scan type ultrasonic endoscope 1 reaches the position to be the reference position.

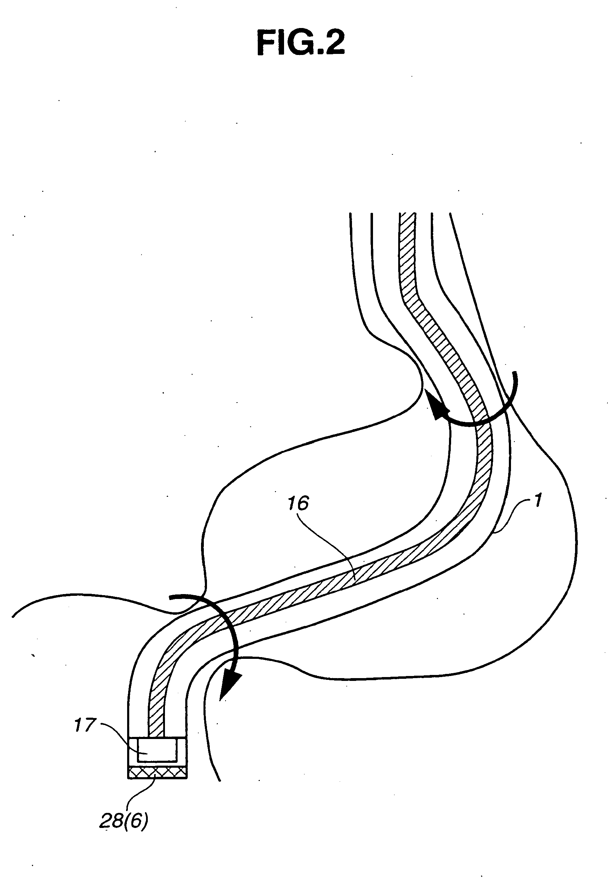

Fig. 2 shows a simplified construction of the mechanical scan type ultrasonic endoscope 1 and a state where the mechanical scan type ultrasonic endoscope 1 is being inserted to the body cavity. As shown in Fig. 2, the mechanical scan type ultrasonic endoscope 1 is fastened at some positions in the body cavity. Under this condition, the ultrasonic transducer 17 at the inserting end is rotated about the shaft 16 so that the shaft 16 is twisted in the mechanical scan type ultrasonic endoscope 1 for scanning ultrasonic wave. As a result, the ultrasonic image may be displaced upward.

A position sensor 28 for detecting the position is provided at the inserting end of the mechanical scan type ultrasonic endoscope 1. Thus, even in the above-described case, the vertical relationship of the screen for scanning ultrasonic wave and schematic diagram data described later can be made in register precisely.

Referring back to Fig. 1, the image synthesizing unit 12 synthesizes the ultrasonic image created by the ultrasonic image creating potion 10 and the schematic diagram data created by the schematic diagram data creating unit 11. Then, the display 3 displays on the same screen the ultrasonic image and the schematic diagram corresponding to the position for scanning ultrasonic wave.

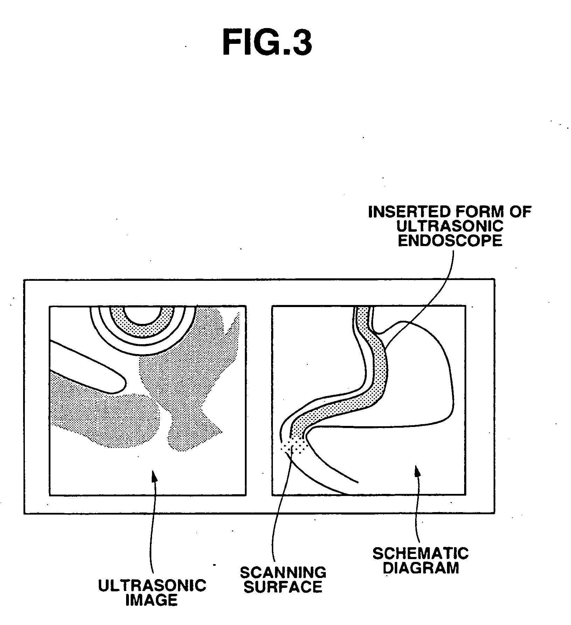

Fig. 3 shows a screen display example. Fig. 3 shows an example displaying the ultrasonic image on the left and the schematic diagram on the right. Like the schematic diagram shown in Fig. 3, the scanning surface of the mechanical scan type ultrasonic endoscope 1 and the inserting form may be displayed together. Alternatively, only the scanning surface or only the schematic diagram without the scanning surface may be displayed.

The schematic diagram on the right of Fig. 3 may be a schema image, a CT image of an object, an MRI image or a human body real optical image obtained from a frozen dead body.

In Fig. 3, the ultrasonic image and the schematic diagram of the scanning position are displayed in alignment. However, the schematic diagram may be displayed over the ultrasonic image.

According to this embodiment, a schematic diagram is displayed together with an ultrasonic image such that the part of an object to be observed can be easily identified. Furthermore, a desired tomography plane can be easily extracted.

### Second Embodiment

A second embodiment is substantially the same as the first embodiment, and only the differences will be described below. The same reference numerals are given to the same components here, and the description will be omitted.

The first embodiment applies a mechanical scan type ultrasonic endoscope but may alternatively apply an ultrasonic endoscope for electrically switching ultrasonic transducers for scanning. The second embodiment will be described below.

Fig. 4 shows a system construction of an ultrasonic diagnosis apparatus according to the second embodiment. Fig. 4 includes an ultrasonic diagnosis apparatus 2, a display 3, an object 4, a position/orientation detecting unit 5, a send coil 6, a receive coil 7, an attitude detecting unit 8, a scope SW 9, an ultrasonic image creating unit 10, a schematic diagram data creating unit 11, an image synthesizing unit 12, a schematic diagram data storing unit 13, a control 14, a keyboard 15, ultrasonic transducers 18, and an electronic radial scan type ultrasonic endoscope 19.

Like the first embodiment, a system construction according to this embodiment uses a magnetic field for position detection.

The ultrasonic diagnosis apparatus 2 according to this embodiment uses the electronic radial scan type ultrasonic endoscope 19 having an array of the ultrasonic transducers 18 including multiple ultrasonic transducers around an inserting axis and includes the send coil 6, which is a position sensor, at the inserting end.

The electronic scan type ultrasonic endoscope 19 electrically switches ultrasonic transducers for transmitting and receiving ultrasonic signals and scans ultrasonic wave on the circumference of the inserting axis. Therefore, like the mechanical scan type ultrasonic endoscope 1 according to the first embodiment, the upward displacement of an ultrasonic image due to the twist of the shaft 16 does not occur. By providing a position sensor at the inserting end, the ultrasonic scan position can be accurately identified. The electronic radial scan type ultrasonic endoscope 19 does not have to have the ultrasonic transducers 18 on the entire circumference of the inserting axis but may be partially lacking, such as in a fan shape of 270 degrees.

Like the first embodiment, the ultrasonic image creating unit 10 creates an ultrasonic image from ultrasonic signals obtained by scanning the ultrasonic transducers 18. Furthermore, the schematic diagram data creating unit 11 detects the attitude of the object 4 obtained from the position/orientation detecting unit 5 and the position for scanning ultrasonic wave of the electronic radial scan type ultrasonic endoscope 19. The schematic diagram data creating unit 11 reads from the schematic diagram data storing unit 13 schematic diagram data corresponding to the position for scanning ultrasonic wave by the electronic radial scan type ultrasonic endoscope 19. Then, the ultrasonic image obtained by the ultrasonic image creating unit 10 and the schematic diagram are displayed on the same screen.

Fig. 5 shows a screen display example. Fig. 5 shows an ultrasonic image by the electronic radial scan type ultrasonic endoscope 19 on the left and a schematic diagram corresponding to the ultrasonic scan position on the right. Like the schematic diagram displayed by the ultrasonic diagnosis apparatus using the mechanical scan type ultrasonic endoscope in Fig. 3, the schematic diagram in Fig. 5 may include the scanning surface and inserting form of the electronic radial scan type ultrasonic endoscope 19. Alternatively, only the scanning plane or the schematic diagram may be included.

Like the first embodiment, according to this embodiment, an ultrasonic image and a schematic diagram are displayed together. Thus, the part being observed of an object can be easily identified, and a desired tomography plane can be easily extracted.

The second embodiment adopts an electronic radial scan type ultrasonic endoscope. Alternatively, the second embodiment may adopt an electronic convex scan type ultrasonic endoscope, which includes an array of ultrasonic transducers and electrically switches transducers.

Fig. 6 shows an electronic radial scan type ultrasonic endoscope. Fig. 7 shows a mechanical scan type ultrasonic endoscope. Fig. 8 shows an electronic convex scan type ultrasonic endoscope. Figs. 6 to 8 show differences between the arrangements and ultrasonic scanning surfaces of the ultrasonic transducers of the ultrasonic endoscopes.

As shown in Fig. 6, the electronic radial scan type ultrasonic endoscope has an array of ultrasonic transducers on the circumference of the inserting axis and ultrasonically scans on the circumference of the inserting axis.

As shown in Fig. 7, the mechanical scan type ultrasonic endoscope mechanically rotates ultrasonic transducers and ultrasonically scans on the circumference of the inserting axis.

As shown in Fig. 8, the electronic convex scan type ultrasonic endoscope has a fan-shaped array of ultrasonic transducers at the end of the inserting axis and ultrasonically scans the surface parallel to the inserting axis.

The electronic convex scan type ultrasonic endoscope scans in the direction different from the scanning direction of the mechanical scan type ultrasonic endoscope and the electronic radial scan type ultrasonic endoscope. However, the electronic convex scan type ultrasonic endoscope may be also applied to the ultrasonic diagnosis apparatus so as to achieve easily-understandable diagnosis.

Fig. 9 shows a display example of an electronic convex scan type ultrasonic endoscope. Fig. 9 shows an ultrasonic image on the left and a schematic diagram corresponding to the ultrasonic scanning position on the right. The scanning surface shown in the schematic diagram on the right is represented differently from the first and second embodiments.

As shown in Fig. 9, the schematic diagram on the right may include the ultrasonic scanning surface and inserting form of the ultrasonic endoscope. Alternatively, only the ultrasonic scanning surface or the schematic diagram may be included.

### Third Embodiment

A third embodiment is substantially the same as the first embodiment. Therefore, only differences will be described. The same reference numerals are given to the same components, and the description will be omitted here.

Since the ultrasonic diagnosis apparatus 2 according to the first and second embodiments has the construction shown in Fig. 10, the name of a part can be displayed over an ultrasonic image in accordance with the ultrasonic scanning. The construction will be described hereinafter.

Fig. 10 shows an ultrasonic diagnosis apparatus 2 according to the third embodiment. The ultrasonic diagnosis apparatus 2 includes an ultrasonic image creating unit 10, a name-of-part superposing unit 20, a name-of-part extracting unit 25, and a display 3. The name-of-part extracting unit 25 according to the third embodiment includes a schematic diagram area extracting unit 21, a reference schematic diagram storing unit 22, a name-of-part storing unit 23 and a name-of-part/area correspondence unit 24.

The ultrasonic image creating unit 10 creates ultrasonic image data from ultrasonic signals obtained by transmitting and receiving ultrasonic wave within an object.

On the other hand, the schematic diagram area extracting unit 21 detects an area of the scanning position of the ultrasonic endoscope from reference schematic diagram data of the reference schematic diagram storing unit 22 based on the signals of the position and direction for detecting the position of the ultrasonic endoscope and the attitude of the object, which have been input to the name-of-part extracting unit 25. Then, the schematic diagram area extracting unit 21 outputs ultrasonic scan area data.

The name-of-part/area correspondence unit 24 reads from the name-of-part storing part 23 name-of-part data corresponding to the output ultrasonic scan area data. The name-of-part superposing part 20 displays on the screen of the display 3 the read name-of-part data over the ultrasonic image.

Fig. 11 shows a screen display example. As shown in Fig. 11, according to this embodiment, in addition to the advantages of the first and second embodiments, a name of a part is superposed on an ultrasonic image. Therefore, the correspondence of the ultrasonic image to an organ becomes clearer, which allows the operator to provide more easily understandable diagnoses.

Furthermore, because of the construction of the name-of-part extracting unit 25 as shown in Fig. 12, the part can be colored. The constructions and operations of variation examples of the name-of-part extracting unit 25 will be described below.

Fig. 12 shows an ultrasonic diagnosis apparatus 2 according to a first variation example of the third embodiment. The ultrasonic diagnosis apparatus 2 includes an ultrasonic image creating unit 10, a name-of-part superposing unit 20, a name-of-part extracting unit 25 and a display 3. The name-of-part extracting unit 25 of the first variation example includes a schematic diagram area extracting unit 21, a reference schematic diagram storing unit 22, a part area reading unit 26, and a part area storing unit 27.

According to the first variation example of the third embodiment, the ultrasonic image creating unit 10 creates ultrasonic image data from ultrasonic signals obtained by transmitting and receiving ultrasonic wave.

On the other hand, like the operation in Fig. 8, the schematic diagram area extracting unit 21 detects an area being scanned by the ultrasonic endoscope from the reference schematic diagram data of the reference schematic diagram storing unit 22 based on the position and direction signals for detecting the position of the ultrasonic endoscope and the attitude of an object, which have been input to the name-of-part extracting unit 25. Then, the ultrasonic scan area data is output.

The part area reading unit 26 reads part area data to be colored in accordance with the ultrasonic scan area data from the part area storing unit 27 based on the ultrasonic scan area data output from the schematic diagram area extracting unit 21. The name-of-part superposing unit 20 superposes and displays the read part area colored data on the ultrasonic image.

Fig. 13 shows a screen display example. As shown in Fig. 13, a part on an ultrasonic image is colored. Therefore, according to the first variation example of the third embodiment, the correspondence of the ultrasonic image to the organ becomes clearer, which allows an operator to provide more easily understandable diagnoses. Furthermore, an operator can provide more easily understandable diagnoses by coloring parts in different colors.

By displaying a part name over the ultrasonic image and the schematic image together, more easily understandable diagnoses can be achived. An ultrasonic diagnosis apparatus implementing the construction will be described below.

Fig. 14 shows an ultrasonic diagnosis apparatus 2 according to a second variation example of the third embodiment. The ultrasonic diagnosis apparatus 2 includes an ultrasonic image creating unit 10, a schematic diagram data creating unit 11, a schematic diagram data storing unit 13, an image creating unit 12, a name-of-part superposing unit 20, a name-of-part extracting unit 25 and a display 3.

According to the second variation example of the third embodiment, the ultrasonic image creating unit 10 creates ultrasonic image data from ultrasonic signals obtained by transmitting and receiving ultrasonic wave.

Signals of the position and direction for scanning ultrasonic wave of the ultrasonic endoscope are input to the name-of-part extracting unit 25, and the name of the part is therefore output. Then, the name-of-part superposing unit 20 superposes the name of the part on the ultrasonic image.

On the other hand, the schematic diagram data creating unit 11 reads schematic diagram data corresponding to the ultrasonic-wave scanning position from the schematic diagram data storing unit 13 based on the input signals of the position and direction for scanning ultrasonic wave of the ultrasonic endoscope. The image synthesizing unit 12 synthesizes the read schematic diagram data and the name-of-part superposed ultrasonic image output from the name-of-part superposing unit 20. Then, the display 3 displays the image on the same screen.

Fig. 15 shows an image display example. An ultrasonic image having a name of a part over an ultrasonic image is displayed on the left while the schematic diagram is displayed on the right. In the schematic diagram on the right as shown in Fig. 15, the ultrasonic-wave scanning surface and the inserting form of the ultrasonic endoscope may be displayed. Alternatively, only the ultrasonic-wave scanning surface or the schematic diagram may be displayed.

### Fourth Embodiment

A fourth embodiment is substantially the same as the first embodiment. Therefore, only the differences will be described. Here, the same reference numerals are given to the same components, and the description will be omitted.

As shown in Fig. 16, the schematic data storing unit 13 according to the fourth embodiment includes schematic diagram data storage devices for types of images including schema images, CT images and real optical human body images obtained from frozen dead bodies. The schematic diagram data storing unit 13 to be referred by the schematic diagram data creating unit 11 may be switched by a switcher 29.

Thus, an operator can read a desired schematic diagram for easily understandable diagnoses.

### Fifth Embodiment

A fifth embodiment is substantially the same as the first embodiment. Therefore, only the differences will be described. The same reference numerals are given to the same components, and the description will be omitted here.

In order to provide a schematic diagram data storing unit, a large amount of capacity is required, which costs a lot. Therefore, as shown in Fig. 17, according to the fifth embodiment, only the minimum schematic diagram data to be used for diagnoses is stored in a minimum schematic diagram data storing unit 30 while the other schematic diagram data is stored in an external backing storage 32. When schematic diagram data to be used for a diagnosis does not exist in the minimum schematic diagram data storing unit 30, the external backing storage 32 is connected to the ultrasonic diagnosis apparatus 2. The schematic diagram data in the minimum schematic diagram data storing unit 30 is updated through a data update control 31.

### Sixth Embodiment

All of the above-described embodiments use a long and narrow, flexible ultrasonic endoscope but the invention may be applied to a case where an egg-shaped capsule type ultrasonic sonde containing a position detecting function as disclosed in Japanese Unexamined Patent Application Publication No. 2000-23980.

Fig. 18 shows an egg-shaped capsule type ultrasonic sonde 33. The capsule type ultrasonic sonde 33 includes a cover member 34, an array transducer 36, a send antenna 39, a battery 38, a magnetic source 37, a transmitter 40, a coil 41, a sending/receiving circuit 35 and a duct 43.

The capsule type ultrasonic sonde 33 transmits and receives ultrasonic wave by driving the array transducer from the sending/receiving circuit 35 by using energy of the battery 38, switches transducers for transmitting and receiving and scans the ultrasonic wave.

Thus, ultrasonic wave is received, is amplified in the sending/receiving circuit 35 and is sent from the send antenna 39 to an external ultrasonic synthesizing operation apparatus (not shown). Then, an ultrasonic image is created.

At the same time, by using the energy of the battery 38, a positional signal is sent from the transmitter 40 through the coil 41 and is received by the receive coil 7 as shown in Fig. 1. Thus, the position/orientation detecting unit 6 detects the position of the capsule type ultrasonic sonde 33.

The subsequent steps for displaying the scanning position over a schematic diagram and for coloring and displaying the name of a part and the part area over an ultrasonic image are performed in accordance with the position of the capsule type ultrasonic sonde 33 like the constructions shown in Figs. 1, 4, 10 and 12.

Apparently, according to the present invention, different embodiments can be variously constructed based on the present invention without departing from the spirit and scope of the present invention. The present invention is only limited by the appended claims and is not limited by the specific embodiments.

### Industrial Applicability

As described above, an ultrasonic diagnosis apparatus according to the present invention is effective for observing concerned parts within a body cavity through ultrasonic tomograms.

## Claims

1. An ultrasonic diagnosis apparatus, comprising:
an ultrasonic wave transmitting/receiving unit (17, 18, 36) for transmitting and receiving ultrasonic waves to or from an object:
an ultrasonic wave scanning position detecting unit (5) for detecting a position of the ultrasonic wave transmitting/receiving unit (17, 18, 36); and
an ultrasonic image creating unit (10) for creating an ultrasonic image based on the ultrasonic signals;
the ultrasonic diagnosis apparatus further comprises a control (14) for obtaining information relating to a part of the object corresponding to position information obtained by the ultrasonic-wave scanning position detecting unit (5) from an anatomical data holding unit (13, 25) having human body anatomical data and displaying the information and the ultrasonic image on the same screen (3); and
**characterized in that** the information relating to a part of the object (4) is a name of the part of the object, and the anatomical data of the human body is a name of the part of the human body and **in that** the ultrasonic diagnosis apparatus further comprises
a schematic diagram data storing unit (13), which is the anatomical data holding unit provided in the ultrasonic diagnosis apparatus, for storing schematic diagram data of a human body; and
a schematic diagram creating unit (11) for reading data from the schematic diagram data storing unit and for creating a schematic diagram corresponding to the position detected by the ultrasonic-wave scanning position detecting unit (5)
wherein the schematic diagram data storing unit further includes: a schematic diagram data switching unit for storing different kinds of multiple pieces of schematic diagram data among human body schema images, object CT images, object MRI images and human body real optical images obtained from frozen dead bodies and for switching the multiple schematic diagram data storing units,
wherein the control (14) further controls the switching by the schematic diagram data switching unit (29)..

2. An ultrasonic diagnosis apparatus according to Claim 1, wherein the information relating to a part of the object is anatomical image information of the part of the object, and the anatomical data of the human body is schematic diagram data of the human body.

3. An ultrasonic diagnosis apparatus according to Claim 1, wherein the anatomical data of the human body is data on a part area of the human body;
wherein the control (14) associates the part of the object with data on a part area of the human body, colors the part corresponding to the part of the ultrasonic image and causes a display to display the colored part.

4. An ultrasonic diagnosis apparatus according to Claim 1,
wherein the human body schematic diagram data is a human body schema image, a CT image of an object, an MRI image of an object or a human body real optical image obtained from a frozen dead body.

5. An ultrasonic diagnosis apparatus according to Claim 1, further comprising:
a position-marked schematic diagram creating unit (11) for creating a position-marked schematic diagram indicating an ultrasonic-wave scanning position by showing schematic diagram data read in accordance with the ultrasonic-wave scanning position over schematic diagram data created by the schematic diagram creating unit.

6. An ultrasonic diagnosis apparatus according to Claim 1,
wherein an ultrasonic image is created from ultrasonic signals obtained by transmitting and receiving ultrasonic wave to or from the inside of the object by using a round-shaped ultrasonic sonde (33) easily swallowable from the mouth cavity of the object.

7. An ultrasonic diagnosis apparatus according to Claim 1,
wherein an ultrasonic image is created from ultrasonic signals obtained by transmitting and receiving ultrasonic wave to or from the inside of the object by using a long and narrow, flexible ultrasonic probe (19) to be inserted to the object;
wherein the ultrasonic probe (19) is:
an electronic radial scan type ultrasonic endoscope having an array of ultrasonic transducers around an inserting axis;
an electronic convex scan type ultrasonic endoscope having ultrasonic transducers in a fan shape at one end of the inserting axis; or
a mechanical scan type ultrasonic endoscope in which an ultrasonic transducer piece rotates about the inserting axis.

8. An ultrasonic diagnosis apparatus, comprising:
an ultrasonic-wave transmitting/receiving unit (17, 18, 36) for transmitting and receiving ultrasonic waves to or from an object;
an ultrasonic-wave scanning position detecting unit (5) for detecting a position of the ultrasonic-wave transmitting/receiving unit (17, 18, 36), and
an ultrasonic image creating unit (10) for creating an ultrasonic image based on the ultrasonic signals,
the ultrasonic diagnosis apparatus further comprises a control (14) for obtaining information relating to a part of the object corresponding to position information obtained by the ultrasonic-wave scanning position detecting unit (5) from an anatomical data holding unit having human body anatomical data and displaying the information and the ultrasonic image on the same screen,
wherein the anatomical data of the human body is data on a part area of the human body; and **characterized in that**
the control (14) associates the part of the object with data on a part area of the human body, colors the part corresponding to the part of the ultrasonic image and causes a display to display the colored part, and **in that** the ultrasonic diagnosis apparatus further comprises
a schematic diagram data storing unit (13), which is the anatomical data holding unit provided in the ultrasonic diagnosis apparatus, for storing schematic diagram data of a human body;
a schematic diagram creating unit (11) for reading data from the schematic diagram data storing unit and for creating a schematic diagram corresponding to the position detected by the ultrasonic-wave scanning position detecting unit (5),
wherein the schematic diagram data storing unit further includes: a schematic diagram data switching unit for storing different kinds of multiple pieces of schematic diagram data among human body schema images, object CT images, object MRI images and human body real optical images obtained from frozen dead bodies and for switching the multiple schematic diagram data storing units,
wherein the control (14) further controls the switching by the schematic diagram data switching unit (29).

## Patentansprüche

1. Ultraschalldiagnosevorrichtung aufweisend:
eine Ultraschallwellenübertragungs-/empfangseinheit (17, 18, 36) zum Übertragen und Empfangen von Ultraschallwellen zu oder von einem Objekt;
eine Ultraschallwellenscanpositions-Erfassungseinheit (5) zum Erfassen einer Position der Ultraschallwellenübertragungs-/empfangseinheit (17, 18, 36); und
eine Ultraschallbilderzeugungseinheit (10) zum Erzeugen eines Ultraschallbilds basierend auf dem Ultraschallsignal,
wobei die Ultraschalldiagnosevorrichtung ferner eine Steuerung (14) zum Erhalten von Informationen bezüglich eines einer Positionsinformation entsprechenden Abschnitts des Objekts aufweist, wobei die Positionsinformation durch die Ultraschallwellenscanpositions-Erfassungseinheit (5) von einer anatomischen Datenhalteeinheit (13, 25) erhalten wird, die anatomische Daten des menschlichen Körpers aufweist und die Informationen und das Ultraschallbild auf dem selben Bildschirm (3) darstellt,
**dadurch gekennzeichnet, dass** die Information bezüglich eines Abschnitts des Objekts (4) ein Name des Abschnitts des Objekts ist, und die anatomischen Daten des menschlichen Körpers ein Name des Abschnitts des menschlichen Körpers sind, und **dadurch**, dass die Ultraschalldiagnosevorrichtung ferner aufweist:
eine Speichereinheit (13) für ein schematisches Diagramm, welche die in der Ultraschalldiagnosevorrichtung vorgesehene anatomische Dateneinheit ist, zum Speichern der schematischen Diagrammdaten eines menschlichen Körpers; und
eine schematische Diagramm-Erzeugungseinheit (11) zum Lesen der Daten von der Speichereinheit für die schematischen Diagrammdaten und zum Erzeugen eines schematischen Diagramms, das der Position entspricht, die von der Ultraschallwellenscanposition-Erfassungseinheit (5) erfasst wurde,
wobei die Speichereinheit für schematische Diagrammdaten ferner umfasst eine schematische Diagrammdatenschalteinheit zum Speichern verschiedener Arten von mehreren Bestandteilen der schematischen Diagrammdaten unter den schematischen Bildern des menschlichen Körpers, Objekt CT-Bilder, Objekt MRI-Bilder und reale optische Bilder des menschlichen Körpers, die von gefrorenen toten Körpern erhalten wurden, und zum Schalten der mehreren Speichereinheiten für schematische Diagrammdaten,
wobei die Steuerung (14) ferner das Schalten durch die schematischen Diagrammschalteinheit (29) steuert.

2. Ultraschalldiagnosevorrichtung nach Anspruch 1, wobei die Information bezüglich eines Abschnitts des Objekts eine anatomische Bildinformation des Abschnitts des Objekts ist und die anatomischen Daten des menschlichen Körpers schematische Diagrammdaten des menschlichen Körpers sind.

3. Ultraschalldiagnosevorrichtung nach Anspruch 1, wobei die anatomischen Daten des menschlichen Körpers Daten eines Teilbereichs des menschlichen Körpers sind, wobei die Steuerung (14) den Abschnitt des Objekts mit Daten eines Teilbereichs des menschlichen Körpers verknüpft, den den Abschnitt des Ultraschallbilds entsprechenden Abschnitt farblich hinterlegt und eine Anzeige zum Darstellen des farblich hinterlegten Abschnitts auslöst.

4. Ultraschalldiagnosevorrichtung nach Anspruch 1,
wobei die schematischen Diagrammdaten des menschlichen Körpers ein schematisches Bild des menschlichen Körpers, ein CT-Bild eines Objekts, ein MRI-Bild eines Objekts oder ein reales optisches Bild des menschlichen Körpers ist, das von einem gefrorenen toten Körper erhalten wurde.

5. Ultraschalldiagnosevorrichtung nach Anspruch 1, ferner aufweisend:
eine positionsmarkierte schematische Diagrammerzeugungseinheit (11) zum Erzeugen eines positionsmarkierten schematischen Diagramms, das eine Ultraschallwellenscanposition durch Anzeigen der schematischen Diagrammdaten anzeigt, wobei die Ultraschallwellenscanposition in Übereinstimmung mit der Ultraschallwellenscanposition über die schematischen Diagrammdaten eingelesen wird, die durch die schematische Diagrammerzeugungseinheit erzeugt wurden.

6. Ultraschalldiagnosevorrichtung nach Anspruch 1,
wobei ein Ultraschallbild durch Ultraschallsignale erzeugt wird, die durch Übertragen und Empfangen von Ultraschallwellen zu und von der Innenseite des Objekts durch Verwenden einer runden Ultraschallsonde (33) erhalten werden, die leicht über die Mundhöhle des Objekts schluckbar ist.

7. Ultraschalldiagnosevorrichtung nach Anspruch 1,
wobei ein Ultraschallbild durch die Ultraschallsignale erzeugt wird, die durch Übertragen und Empfangen der Ultraschallwellen zu oder von der Innenseite des Objekts durch Verwendung einer langen, schmalen, flexiblen Ultraschallsonde (19) erhalten werden, die in das Objekt einführbar ist,
wobei die Ultraschallsonde (19):
ein Ultraschallendoskop vom elektronischen Radialscantyp mit einer Reihe von Ultraschallwandlern um eine Einführachse ist;
ein Ultraschallendoskop vom elektronischen Konvexscantyp mit Ultraschallwandlern in einer Flügelform an einem Ende der Einführachse ist; oder
ein Ultraschallendoskop vom mechanischen Scantyp ist, in dem ein Ultraschallwandlerstück um die Einführachse rotiert.

8. Ultraschalldiagnosevorrichtung, aufweisend:
eine Ultraschallwellenübertragungs-/empfangseinheit (17, 18, 36) zum Übertragen und Empfangen von Ultraschallwellen zu oder von einem Objekt;
eine Ultraschallwellenscanpositions-Erfassungseinheit (5) zum Erfassen einer Position der Ultraschallwellenübertragungs-/empfangseinheit (17, 18, 36), und
eine Ultraschallbilderzeugungseinheit (10) zum Erzeugen eines Ultraschallbilds basierend auf den Ultraschallsignalen,
wobei die Ultraschalldiagnosevorrichtung ferner eine Steuerung (14) zum Erhalten von Informationen bezüglich eines Abschnitts des Objekts aufweist, der einer Positionsinformation entspricht, die durch die Ultraschallwellenscanpositions-Erfassungseinheit (5) von einer anatomischen Datenhalteeinheit erhalten wird, die anatomische Daten des menschlichen Körpers umfasst und die Information und das Ultraschallbild auf demselben Bildschirm darstellt,
wobei die anatomischen Daten des menschlichen Körpers Daten eines Teilbereichs des menschlichen Körpers sind, **dadurch gekennzeichnet, dass**
die Steuerung (14) den Abschnitt des Objekts mit Daten auf einem Teilbereich des menschlichen Körpers verknüpft, den dem Abschnitt des Ultraschallbilds entsprechenden Abschnitt farblich hinterlegt und ein Display zum Darstellen des farblich hinterlegten Abschnitts anweist, und **dadurch**, dass die Ultraschalldiagnosevorrichtung ferner aufweist:
eine Speichereinheit (13) für schematische Diagrammdaten, die die in der Ultraschalldiagnosevorrichtung vorgesehene anatomische Datenhalteeinheit ist, zum Speichern der schematischen Diagrammdaten des menschlichen Körpers;
eine schematische Diagrammerzeugungseinheit (11) zum Auslesen der Daten aus der Speichereinheit für die schematischen Diagrammdaten und zum Erzeugen eines schematischen Diagramms, das der durch die Ultraschallwellenscanpositions-Erfassungseinheit (5) erfassten Position entspricht,
wobei die Speichereinheit für schematische Diagrammdaten ferner umfasst:
eine schematische Diagrammdatenschalteinheit zum Speichern verschiedener Arten von mehreren Bestandteilen von schematischen Diagrammdaten unter den schematischen Bildern des menschlichen Körpers, CT-Bildern des Objekts, MRI-Bildern des Objekts und realen optischen Bildern des menschlichen Körpers, die von gefrorenen toten Körpern erhalten wurden, und zum Schalten der mehreren schematischen Diagrammdatenspeichereinheiten,
wobei die Steuerung (14) ferner das Schalten durch die schematische Diagrammdatenschalteinheit (29) steuert.

## Revendications

1. Appareil de diagnostic ultrasonore, comprenant :
une unité d'émission/réception d'ondes ultrasonores (17, 18, 36) pour émettre et recevoir des ondes ultrasonores vers un ou d'un objet ;
une unité de détection de position de balayage d'ondes ultrasonores (5) pour détecter une position de l'unité d'émission/réception d'ondes ultrasonores (17, 18, 36) ; et
une unité de création d'image ultrasonore (10) pour créer une image ultrasonore sur la base des signaux ultrasonores ;
l'appareil de diagnostic ultrasonore comprend en outre une commande (14) pour obtenir une information se rapportant à une partie de l'objet correspondant à l'information de position obtenue par l'unité de détection de position de balayage d'ondes ultrasonores (5) à partir d'une unité de conservation de données anatomiques (13, 25) ayant des données anatomiques du corps humain et afficher l'information et l'image ultrasonore sur le même écran (3) ; et
**caractérisé en ce que** l'information se rapportant à une partie de l'objet (4) est un nom de la partie de l'objet, et les données anatomiques du corps humain sont un nom de la partie du corps humain et **en ce que** l'appareil de diagnostic ultrasonore comprend en outre
une unité de stockage de données de diagramme schématique (13), qui est l'unité de conservation de données anatomiques prévue dans l'appareil de diagnostic ultrasonore, pour stocker des données de diagramme schématique d'un corps humain ; et
une unité de création de diagramme schématique (11) pour lire des données à partir de l'unité de stockage de données de diagramme schématique et pour créer un diagramme schématique correspondant à la position détectée par l'unité de détection de position de balayage d'ondes ultrasonores (5)
dans lequel l'unité de stockage de données de diagramme schématique inclut en outre : une unité de commutation de données de diagramme schématique pour stocker différents types de parties multiples de données de diagramme schématique parmi des images de schéma du corps humain, des images de TDM d'objet, des images d'IRM d'objet et des images optiques réelles du corps humain obtenues à partir de cadavres congelés et pour commuter les unités de stockage de données de diagramme schématique multiples,
dans lequel la commande (14) commande en outre la commutation par l'unité de commutation de données de diagramme schématique (29).

2. Appareil de diagnostic ultrasonore selon la revendication 1, dans lequel l'information se rapportant à une partie de l'objet est une information d'image anatomique de la partie de l'objet, et les données anatomiques du corps humain sont des données de diagramme schématique du corps humain.

3. Appareil de diagnostic ultrasonore selon la revendication 1, dans lequel les données anatomiques du corps humain sont des données sur un zone partielle du corps humain ;
dans lequel la commande (14) associe la partie de l'objet avec des données sur une zone partielle du corps humain, colore la partie correspondant à la partie de l'image ultrasonore et fait en sorte qu'un afficheur affiche la partie colorée.

4. Appareil de diagnostic ultrasonore selon la revendication 1,
dans lequel les données de diagramme schématique du corps humain sont une image de schéma du corps humain, une image de TDM d'un objet, une image d'IRM d'un objet ou une image optique réelle du corps humain obtenue à partir d'un cadavre congelé.

5. Appareil de diagnostic ultrasonore selon la revendication 1, comprenant en outre :
une unité de création de diagramme schématique à marque de position (11) pour créer un diagramme schématique à marque de position indiquant une position de balayage d'ondes ultrasonores en montrant des données de diagramme schématique lues en fonction de la position de balayage d'ondes ultrasonores par-dessus des données de diagramme schématique créées par l'unité de création de diagramme schématique.

6. Appareil de diagnostic ultrasonore selon la revendication 1,
dans lequel une image ultrasonore est créée à partir de signaux ultrasonores obtenus en émettant et en recevant une onde ultrasonore vers ou de l'intérieur de l'objet en utilisant une sonde ultrasonore (33) de forme arrondie facilement avalable par la cavité buccale de l'objet.

7. Appareil de diagnostic ultrasonore selon la revendication 1,
dans lequel une image ultrasonore est créée à partir de signaux ultrasonores obtenus en émettant et en recevant une onde ultrasonore vers ou de l'intérieur de l'objet en utilisant une sonde ultrasonore (19) flexible, longue et étroite destinée à être insérée dans l'objet ;
dans lequel la sonde ultrasonore (19) est :
un endoscope ultrasonore de type à balayage radial électronique ayant une matrice de transducteurs ultrasonores autour d'un axe d'insertion ;
un endoscope ultrasonore de type à balayage convexe électronique ayant des transducteurs ultrasonores en une forme d'éventail à une extrémité de l'axe d'insertion ; ou
un endoscope ultrasonore de type à balayage mécanique dans lequel un transducteur ultrasonore tourne autour de l'axe d'insertion.

8. Appareil de diagnostic ultrasonore, comprenant :
une unité d'émission/réception d'ondes ultrasonores (17, 18, 36) pour émettre et recevoir des ondes ultrasonores vers un ou depuis un objet ;
une unité de détection de position de balayage d'ondes ultrasonores (5) pour détecter une position de l'unité d'émission/réception d'ondes ultrasonores (17, 18, 36) ; et
une unité de création d'image ultrasonore (10) pour créer une image ultrasonore sur la base des signaux ultrasonores,
l'appareil de diagnostic ultrasonore comprend en outre une commande (14) pour obtenir une information se rapportant à une partie de l'objet correspondant à l'information de position obtenue par l'unité de détection de position de balayage d'ondes ultrasonores (5) à partir d'une unité de conservation de données anatomiques ayant des données anatomiques du corps humain et afficher l'information et l'image ultrasonore sur le même écran,
dans lequel les données anatomiques du corps humain sont des données sur une zone partielle du corps humain ; et **caractérisé en ce que**
la commande (14) associe la partie de l'objet avec des données sur une zone partielle du corps humain, colore la partie correspondant à la partie de l'image ultrasonore et fait en sorte qu'un afficheur affiche la partie colorée, et **en ce que** l'appareil de diagnostic ultrasonore comprend en outre
une unité de stockage de données de diagramme schématique (13), qui est l'unité de conservation de données anatomiques prévue dans l'appareil de diagnostic ultrasonore, pour stocker des données de diagramme schématique d'un corps humain ;
une unité de création de diagramme schématique (11) pour lire des données à partir de l'unité de stockage de données de diagramme schématique et pour créer un diagramme schématique correspondant à la position détectée par l'unité de détection de position de balayage d'ondes ultrasonores (5),
dans lequel l'unité de stockage de données de diagramme schématique inclut en outre : une unité de commutation de données de diagramme schématique pour stocker différents types de parties multiples de données de diagramme schématique parmi des images de schéma du corps humain, des images de TDM d'objet, des images d'IRM d'objet et des images optiques réelles du corps humain obtenues à partir de cadavres congelés et pour commuter les unités de stockage de données de diagramme schématique multiples,
dans lequel la commande (14) commande en outre la commutation par l'unité de commutation de données de diagramme schématique (29).
